(19) **Europäisches Patentamt European Patent Office Office européen des brevets**

(11) **EP 4 255 338 B1**

(12) **FASCICULE DE BREVET EUROPEEN**

(45) Date de publication et mention de la délivrance du brevet:
**25.12.2024 Bulletin 2024/52**

(51) Classification Internationale des Brevets (IPC):
*A61C 5/50* *(2017.01)* *A61K 6/858* *(2020.01)*
*A61P 1/02* *(2006.01)* *A61P 19/08* *(2006.01)*
*A61P 29/00* *(2006.01)* *A61P 31/04* *(2006.01)*
*A61P 31/12* *(2006.01)*

(21) Numéro de dépôt: **21819930.5**

(22) Date de dépôt: **15.11.2021**

(52) Classification Coopérative des Brevets (CPC):
**A61C 5/50; A61K 6/54; A61K 6/858; A61P 1/02; A61P 19/08; A61P 29/00; A61P 31/04; A61P 31/12**

(86) Numéro de dépôt international:
**PCT/FR2021/052017**

(87) Numéro de publication internationale:
**WO 2022/117929 (09.06.2022 Gazette 2022/23)**

(54) **PRODUIT DENTAIRE POUR LA FORMATION D'UN CIMENT ENDODONTIQUE**

DENTALPRODUKT ZUR HERSTELLUNG EINES ENDODONTISCHEN ZEMENTS

DENTAL PRODUCT FOR FORMING AN ENDODONTIC CEMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **02.12.2020 FR 2012532**

(43) Date de publication de la demande:
**11.10.2023 Bulletin 2023/41**

(73) Titulaire: **Produits Dentaires Pierre Rolland**
**33700 Merignac (FR)**

(72) Inventeurs:
• **MAURAT, Vincent**
 **33600 Pessac (FR)**
• **LANDRODIE, Marine**
 **24600 Riberac (FR)**

(74) Mandataire: **Cabinet Beau de Loménie**
 **158, rue de l'Université**
 **75340 Paris Cedex 07 (FR)**

(56) Documents cités:
**WO-A2-2005/115488 WO-A2-2007/047994**
**WO-A2-2008/102214 FR-A1- 3 047 414**
**US-A1- 2005 079 470**

**Description**

Domaine Technique

L'invention concerne un produit dentaire permettant la formation d'un ciment endodontique, ou ciment de scellement canalaire, en présence d'humidité dans un canal dentaire. Le ciment formé est apte à subir une opération de reprise aisée, par exemple par application d'ultrasons.

Technique antérieure

Le traitement endodontique est une intervention délicate sur laquelle repose la santé parodontale et la pérennité de l'organe dentaire. Il consiste en la prévention, le diagnostic et le traitement de la pulpe dentaire et des infections péri-apicales (dans l'os et autour des racines) afin de transformer une dent pathologique en une entité saine, asymptomatique et fonctionnelle sur l'arcade.

Le traitement canalaire ou endodontique est indiqué dans le cadre d'une pulpite irréversible (inflammation, infection pulpaire) ou d'une pulpe nécrosée avec ou sans signe clinique et/ou radiographique de parodontite apicale (lésions inflammatoires du parodonte) ou encore dans le cadre d'une pulpe vivante si le pronostic de vitalité pulpaire est défavorable, si présence d'une amputation radiculaire ou hémisection et si la probabilité d'exposition pulpaire au cours de la restauration coronaire n'autorisant pas le coiffage direct est élevée.

L'inflammation et l'infection pulpaire peuvent être dues à de multiples causes comme les caries, fêlures, fracture ou choc dentaire. Si la pulpe infectée ou inflammée n'est pas soignée, elle peut causer d'importantes douleurs et même conduire à un abcès. Le traitement endodontique consiste à prévenir ou supprimer l'infection, par l'élimination des bactéries et de leurs toxines du système canalaire, mais aussi de tous les résidus susceptibles de servir de nutriments et de support à la multiplication bactérienne. Afin de maintenir cette désinfection, il faut remplacer la pulpe ou ce qu'il en reste, dans un système canalaire sain, par une obturation étanche, favorisant la cicatrisation. Cet acte permet l'élimination et la neutralisation de substances organiques et bactériennes qui sont à l'origine d'irritation des tissus péri-apicaux. Le résultat doit être stable et durable. Une fois reconstituée, la dent doit être fonctionnelle, asymptomatique et ne présenter aucun signe clinique.

On connaît l'emploi de ciments de scellement canalaire qui sont bien tolérés et présentant une bonne tenue. Néanmoins, l'opération de retraitement du ciment dentaire posé au préalable en cas de besoin de reprise du traitement endodontique, par exemple pour restaurer le ciment, peut être délicate. Il peut, en effet, s'avérer nécessaire d'utiliser des solvants, potentiellement toxiques, pour réduire la dureté des ciments au niveau du canal pour l'opération de reprise. On connaît WO 2008/102214 qui divulgue un matériau de remplissage endodontique composé d'un mélange de poudres de Phosphate de Calcium, Silicate de Calcium et de sels de Calcium dans une solution aqueuse.

Il est souhaitable de disposer de produits dentaires pour la formation d'un ciment endodontique qui produise l'obturation canalaire souhaitée tout en rendant plus aisée une opération de reprise.

Exposé de l'invention

L'invention concerne un produit dentaire destiné à former un ciment endodontique dans un canal dentaire, ledit produit comprenant au moins :

- une composition pulvérulente en une teneur massique comprise entre 40 % et 55 % comprenant au moins un phosphate de calcium et apte à former de l'hydroxyapatite en présence d'humidité,
- un liant en une teneur massique comprise entre 0,5 % et 5 %, et
- un milieu liquide en une teneur massique comprise entre 30% et 50%.

La composition particulière du produit dentaire décrite ci-dessus confère plusieurs avantages au ciment endodontique obtenu.

En effet, ce ciment est biocompatible et non irritant. Il est à base d'hydroxyapatite, qui est le constituant majoritaire de la dentine, et sa prise s'effectue naturellement par mise en contact du produit avec l'humidité canalaire. Le ciment obtenu présente en outre une cohésion suffisante conférant la tenue souhaitée sans que celle-ci ne soit trop importante pour qu'une opération de reprise aisée et sécuritaire, par exemple à la pointe ultrasonore, soit possible. Ainsi, le ciment présente avantageusement une résistance à la compression comprise entre 3 MPa et 15 MPa, par exemple comprise entre 3 MPa et 10 MPa. La viscosité du produit est en outre limitée afin qu'il puisse être facilement introduit dans le canal dentaire sans formation de bulle d'air. Ainsi, la viscosité dynamique du produit est avantageusement inférieure ou égale à 240 Pa.s, par exemple inférieure ou égale à 200 Pa.s, par exemple inférieure ou égale à 150 Pa.s. La viscosité peut être déterminée par un rhéomètre plan-plan, par exemple commercialisé sous la référence « Discovery HR-2 » de la société TA instruments, à 20°C. On peut utiliser une méthode « rampe d'écoulement continu » (« Flow ramp ») de de 0,01 à 10 s$^{-1}$ et une méthode « balayage en écoulement par paliers » (« Flow sweep ») de 10 à 1.10$^{-6}$ s$^{-1}$ pour mesurer la viscosité et le seuil d'écoulement du produit dentaire.

Plus précisément, la teneur en composition pulvérulente est suffisante pour que la résistance à la compression du ciment soit suffisante pour obtenir la cohésion souhaitée tout en restant limitée pour permette l'opération de reprise et que le produit conserve une viscosité maîtrisée. Le milieu liquide, présent dans le produit en une teneur significative, participe aussi à limiter la viscosité. La teneur en liant est suffisante pour participer à obtenir la cohésion souhaitée tout en restant limitée de sorte à ne pas affecter la viscosité.

On notera que le produit dentaire ayant la composition décrite ci-dessus correspond au produit prêt à être introduit dans le canal dentaire pour formation du ciment. Le produit peut être conditionné en une seule et même composition comprenant l'ensemble de ses constituants avant son utilisation ou en plusieurs parties séparées, chacune dans un compartiment distinct, destinées à être mélangées seulement au moment de l'utilisation du produit, comme il sera décrit plus en détails dans la suite. Sauf mention contraire, les teneurs des constituants du produit dentaire sont prises avant introduction dans le canal dentaire.

Dans un exemple de réalisation, la teneur massique de la composition pulvérulente est comprise entre 43 % et 52 %.

Une telle caractéristique participe avantageusement à optimiser le compromis entre la résistance à la compression du ciment et la viscosité du produit.

Dans un exemple de réalisation, la composition pulvérulente comprend un mélange de phosphate dicalcique et de phosphate tétracalcique.

En particulier, le rapport entre la masse de phosphate tétracalcique dans le produit et la masse de phosphate dicalcique dans le produit peut être compris entre 0,9 et 1,9.

Dans un exemple de réalisation, la composition pulvérulente a une taille moyenne D50 de grains inférieure ou égale à 50 μm.

Par « taille moyenne D50 », on désigne la dimension donnée par la distribution granulométrique statistique à la moitié de la population.

Le choix de grains fins pour la composition pulvérulente participe à obtenir une cohésion améliorée ainsi qu'une prise rapide du ciment dans le canal dentaire. Dans un exemple de réalisation, la teneur massique du liant est comprise entre 0,5 % et 3,5 %.

Une telle caractéristique participe avantageusement à optimiser le compromis entre la résistance à la compression du ciment et la viscosité du produit.

Dans un exemple de réalisation, le produit comprend en outre un agent de contraste radio-opaque en une teneur massique inférieure ou égale à 15%.

La présence de l'agent de contraste radio-opaque permet le suivi et le contrôle radiographique du ciment lors de sa pose. Lorsqu'il est présent, sa teneur doit néanmoins rester limitée afin de ne pas altérer la résistance à la compression du ciment en affectant la formation du réseau par les phosphates de calcium.

Dans un exemple de réalisation, le produit comprend en outre un agent de suspension en une teneur massique inférieure ou égale à 1,5%.

La présence de l'agent de suspension permet d'éviter tout risque de séparation entre la phase solide et la phase liquide dans le produit. Lorsqu'il est présent, sa teneur doit néanmoins rester limitée afin de ne pas trop augmenter la viscosité du produit. Dans un exemple de réalisation, le produit comprend en outre un accélérateur de prise du ciment en une teneur massique inférieure ou égale à 10%.

Ce composé permet d'accélérer la formation du ciment. Lorsqu'il est présent, sa teneur doit néanmoins rester limitée afin de ne pas trop augmenter la viscosité du produit.

Dans un exemple de réalisation, le produit est tel que :

- la teneur massique en composition pulvérulente est comprise entre 43% et 52%,
- la teneur massique en liant est comprise entre 0,5% et 1%,
- le milieu liquide est non-aqueux et sa teneur massique est comprise entre 30% et 40%,
- le produit comprend un agent de contraste radio-opaque en une teneur massique comprise entre 5% et 15%, par exemple entre 5% et 12%, et
- le produit comprend un accélérateur de prise du ciment en une teneur massique comprise entre 4% et 7%.

En particulier, l'agent de contraste radio-opaque peut comprendre du sulfate de baryum et la teneur massique en composition pulvérulente peut être comprise entre 43% et 48%.

En variante, l'agent de contraste radio-opaque peut comprendre de l'oxyde de zirconium et la teneur massique en composition pulvérulente peut être comprise entre 48% et 52%.

Selon un autre exemple de réalisation, le produit est tel que :

- la teneur massique en composition pulvérulente est comprise entre 43% et 52%,
- la teneur massique en liant est comprise entre 3% et 3,5%,
- le milieu liquide comprend de l'eau et sa teneur massique est comprise entre 30% et 40%, et
- le produit comprend un agent de contraste radio-opaque en une teneur massique comprise entre 5% et 15 %.

L'invention vise également un ensemble pour l'obturation et le scellement d'un canal dentaire, comprenant au moins le produit tel que décrit plus haut dans un dispositif d'introduction apte à introduire le produit dans le canal dentaire.

L'invention vise également un procédé de pose d'un ciment endodontique comprenant au moins :

- l'introduction d'un produit dentaire tel que décrit plus haut dans un canal dentaire, et
- la prise du produit dentaire ainsi introduit au contact de l'humidité présente dans le canal dentaire afin de former le ciment endodontique.

L'invention vise également un procédé de traitement d'un ciment endodontique obtenu à partir du produit dentaire tel que décrit plus haut et présent dans un canal dentaire dans lequel on met en contact ledit ciment endodontique avec un outil vibrant à une fréquence ultrasonore.

Brève description des dessins

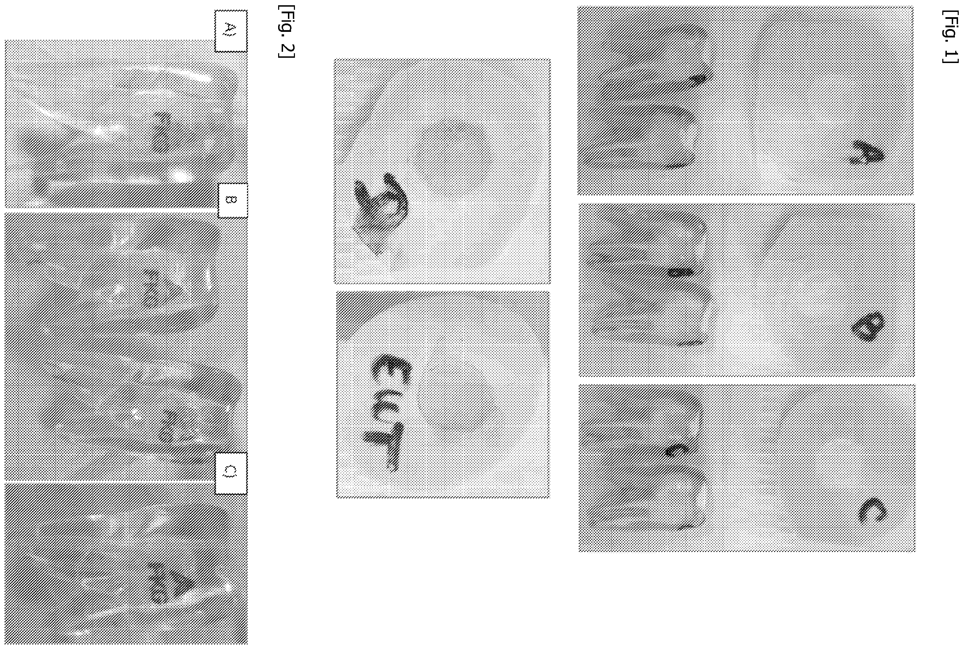

[Fig. 1] La figure 1 fournit des photographies d'éprouvettes et de modèles de dents remplis de différents ciments canalaires.

[Fig. 2] La figure 2 fournit des photographies de modèles de dents après retraitement du ciment canalaire.

Description des modes de réalisation

Le produit dentaire est sous forme fluide et comprend au moins la composition pulvérulente et le liant dans le milieu liquide. Le produit dentaire peut être sous la forme d'une suspension. Le produit dentaire est destiné à être introduit dans un canal dentaire pour former un ciment endodontique de remplissage et scellement de ce canal. La viscosité dynamique du produit dentaire peut être comprise entre 90 Pa.s et 240 Pa.s, par exemple entre 90 Pa.s et 200 Pa.s, voire entre 90 Pa.s et 150 Pa.s. Comme indiqué plus haut, la résistance à la compression du ciment obtenu peut être comprise entre 3 MPa et 15 MPa, par exemple comprise entre 3 MPa et 10 MPa.

La résistance à la compression du ciment peut être mesurée par mise en oeuvre du protocole opératoire décrit ci-dessous. Un moule en plâtre de Paris de 6 mm de diamètre et 10 mm de hauteur permettant un échange en eau a été rempli du produit dentaire à l'aide d'une spatule en prenant soin de ne pas laisser de bulles d'air. Le moule rempli a été placé dans une étuve à 40°C et haute humidité relative pendant 3 jours. Après 3 jours, l'éprouvette en ciment endodontique a été démoulée en évitant de l'endommager en la soumettant à des forces trop importantes. L'éprouvette a ensuite été soumise à un essai de résistance à la compression à l'aide de la machine d'essai universelle commercialisée sous la référence AGS-X par la société SHIMADZU équipée de la cellule 500 N ou 10 kN, du support mobile et du mobile P/25. Une vitesse de test de 0,750 mm/minute a été imposée durant le test en compression jusqu'à rupture de l'éprouvette. La résistance à la compression Rc est alors déterminée à l'aide de la formule suivante où Fmax désigne la force appliquée juste avant la rupture de l'éprouvette et d le diamètre initial de l'éprouvette.

[Math. 1]

$$Rc = \frac{Fmax}{(\pi x d^2)/4}$$

La suite s'attache à apporter plus de détails sur les différents constituants du produit dentaire. Les différentes caractéristiques qui vont être décrites plus bas peuvent être prises isolément ou être combinées entre elles.

Le produit dentaire comprend une composition pulvérulente qui est apte à former de l'hydroxyapatite $Ca_{10}(PO_4)_6(OH)_2$ en présence d'humidité, notamment au contact de l'humidité présente dans le canal dentaire dans lequel le produit est introduit, afin de former le ciment dentaire.

La composition pulvérulente comprend au moins un phosphate de calcium et peut, selon un exemple, comprendre un mélange d'un phosphate de calcium acide et d'un phosphate de calcium basique qui sont aptes à réagir en présence d'humidité pour former l'hydroxyapatite. Dans ce cas, les phosphates de calcium acide et basique sont chacun sous la forme d'une poudre.

La formation d'hydroxyapatite à partir d'au moins un phosphate de calcium est connue en soi. On peut citer l'emploi d'un mélange de phosphate dicalcique, qui peut être anhydre de formule $CaHPO_4$ ou dihydraté de formule $CaHPO_4, 2 H_2O$, et de phosphate tétracalcique de formule $Ca_4(PO_4)_2O$. Le phosphate dicalcique est un phosphate de calcium acide et le phosphate tétracalcique un phosphate de calcium basique. On peut encore utiliser une composition pulvérulente comprenant un mélange d'un phosphate de calcium, comme le phosphate dicalcique dihydraté, avec du carbonate de calcium ou de l'hydroxyde de calcium. Néanmoins, on utilisera préférentiellement dans le cadre de l'invention une composition pulvérulente comprenant, par exemple constituée essentiellement de, un mélange d'un phosphate de calcium acide, comme le phosphate dicalcique, et d'un phosphate de calcium basique, comme le phosphate tétracalcique. On peut par exemple utiliser un mélange équimolaire de phosphate dicalcique et de phosphate tétracalcique qui réagissent en présence d'humidité pour former l'hydroxyapatite. La composition pulvérulente peut, selon un exemple, être dépourvue de carbonate de calcium, d'hydroxyde de calcium ou de silicate tricalcique.

La taille moyenne D50 de grains de la composition pulvérulente peut être inférieure ou égale à 50 μm, comme indiqué plus haut. Selon un exemple, lorsque l'on utilise un mélange de phosphate de calcium acide et de phosphate de calcium basique, la taille moyenne D50 de grains du phosphate de calcium basique peut être inférieure ou égale à 10 μm. Une telle caractéristique participe avantageusement à accélérer la prise du ciment. Comme indiqué plus haut, la composition pulvérulente est présente dans le produit dentaire en une teneur massique comprise entre 40% et 55%, par exemple entre 43% et 52%, par exemple entre 45% et 50%. Dans le cas où la composition pulvérulente comprend un mélange de phosphate dicalcique et de phosphate tétracalcique, le rapport massique [masse de phosphate tétracalcique dans le produit] / [masse de phosphate dicalcique dans le produit] peut être compris entre 0,9 et 1,9.

Le produit dentaire comprend un milieu liquide dans lequel la composition pulvérulente et le liant sont présents. Le milieu liquide peut comporter l'un au moins des composés suivants : glycérol, propylène glycol, eau et leurs mélanges. Le milieu liquide peut être non-aqueux ou, en variante, comprendre de l'eau. Il a été indiqué plus haut que le produit dentaire peut être conditionné en une seule et même composition comprenant l'ensemble de ses constituants avant son utilisation. Dans ce cas, on privilégiera l'emploi d'un milieu liquide non-aqueux afin d'éviter la formation d'hydroxyapatite avant l'utilisation. Selon une variante, le produit est conditionné en plusieurs parties séparées, chacune dans un compartiment distinct, destinées à être mélangées seulement au moment de l'utilisation du produit. Dans ce cas, on peut par exemple avoir une composition pulvérulente comprenant un mélange d'un phosphate de calcium acide et d'un phosphate de calcium basique et conditionner le phosphate de calcium acide dans un premier compartiment et le phosphate de calcium basique dans un deuxième compartiment, séparé du premier compartiment. Dans ce cas, on peut utiliser de l'eau dans le premier compartiment et/ou le deuxième compartiment, étant donné qu'avant l'utilisation les phosphates de calcium acide et basique sont séparés et ne sont pas en mesure de réagir pour former l'hydroxyapatite. Comme indiqué plus haut, le milieu liquide est présent dans le produit dentaire en une teneur massique comprise entre 30% et 50%, par exemple entre 30% et 40%.

Le produit dentaire comprend en outre un liant qui est formé d'un composé distinct de celui ou ceux formant la composition pulvérulente. Selon un exemple, le liant peut être choisi parmi : la carboxyméthylcellulose, la polyvinylpyrrolidone, et les mélanges de ces composés. On peut par exemple utiliser le produit commercialisé sous la référence Kollidon® 90 F par la société BASF. Comme indiqué plus haut, le liant est présent dans le produit dentaire en une teneur massique comprise entre 0,5% et 5%, par exemple entre 0,5% et 3,5%. Plus particulièrement dans le cas où le milieu liquide comprend de l'eau, la teneur massique en liant dans le produit dentaire peut être comprise entre 3% et 5%, par exemple entre 3% et 3,5%. En variante, lorsque le milieu liquide est non-aqueux, la teneur massique en liant dans le produit dentaire peut être comprise entre 0,5% et 1%.

Comme indiqué plus haut, le produit dentaire peut comporter d'autres constituants dans le milieu liquide en plus de la composition pulvérulente et du liant. Ces constituants supplémentaires éventuellement présents sont décrits dans la suite.

Le produit dentaire peut comporter en outre un agent de contraste radio-opaque lequel peut être choisi parmi : le sulfate de baryum, l'oxyde de zirconium, ou les mélanges de ces composés. L'agent de contraste radio-opaque peut être présent dans le produit dentaire en une teneur massique inférieure ou égale à 15%, par exemple comprise entre 5% et 14%, par exemple entre 5% et 12%.

Comme indiqué plus haut, le produit dentaire peut comporter en outre un agent de suspension. L'agent de suspension peut comporter de la silice. On peut par exemple utiliser le produit commercialisé sous la référence Aerosil® 200 par la société Evonik. L'agent de suspension peut être présent dans le produit dentaire en une teneur massique inférieure ou égale à 1,5%, par exemple inférieure ou égale à 1%. Cette teneur peut être comprise entre 0,25% et 1,5% par exemple entre 0,25% et 1%, ou entre 0,5% et 1,5% par exemple entre 0,5% et 1%.

Comme indiqué plus haut, le produit dentaire peut comporter en outre un accélérateur de prise du ciment.

L'accélérateur de prise peut être de l'hydroxyapatite, du phosphate de sodium, ou un mélange de ces composés. L'accélérateur de prise peut être présent dans le produit dentaire en une teneur massique inférieure ou égale à 10%, par exemple inférieure ou égale à 7% ou inférieure ou égale à 6%. Cette teneur peut être comprise entre 4% et 10%, par exemple entre 4% et 7%, par exemple entre 5% et 6%.

Le produit dentaire peut en outre comporter au moins un conservateur. Le conservateur peut être présent dans le produit dentaire en une teneur massique inférieure ou égale à 1,5%, par exemple inférieure ou égale à 1%. Cette teneur massique peut être comprise entre 0,15% et 1,5% par exemple entre 0,15% et 1%, ou entre 0,5% et 1,5%, par exemple entre 0,5% et 1%. Le conservateur permet la stabilité du produit dentaire dans le temps et peut être choisi parmi : le méthyl parabène, le propyl parabène, la chlorhexidine, et les mélanges de ces composés. Le produit dentaire peut comporter au moins un agent de traitement dentaire, par exemple un agent thérapeutique, comme l'un au moins d'un antiseptique ou d'un anti-inflammatoire. L'agent antiseptique peut être choisi parmi le chlorhydrate de chlorhexidine, le dichlorhydrate de chlorhexidine, le gluconate de chlorhexidine, le digluconate de chlorhexidine, l'hypochlorite de sodium, les ammoniums quaternaires, les dérivés iodés ou leurs mélanges. L'agent antiseptique peut en particulier comprendre du gluconate de chlorhexidine ou du digluconate de chlorhexidine. L'agent anti-inflammatoire peut être choisi parmi le butoforme, l'acétate de prednisolone, l'acide β-glycyrrhétinique, un anti-inflammatoire non stéroïdien ou leurs mélanges.

On vient de décrire différents détails relatifs à la composition du produit dentaire. La suite fournit des détails sur l'utilisation de ce produit en vue de former le ciment dentaire et procéder à son retraitement.

Le produit dentaire peut être conditionné en une seule et même composition comprenant l'ensemble de ses constituants avant son utilisation ou en plusieurs parties séparées, chacune dans un compartiment distinct, destinées à être mélangées seulement au moment de l'utilisation du produit dentaire, avec par exemple séparation des phosphates de calcium acide et basique.

Le dispositif d'introduction permettant d'introduire le produit dentaire dans le canal radiculaire peut être une seringue simple ou double compartiment avec des embouts spécifiques pour le remplissage du canal. Un tel dispositif d'introduction est connu en soi et à titre d'exemple de dispositif d'introduction utilisable, on peut citer l'outil commercialisé sous la référence Colibri™ par la société Sulzer Mixpac™ (exemple double compartiement). Une fois introduit dans le canal radiculaire, le produit dentaire durcit au contact de l'humidité dans le canal afin de former de l'hydroxyapatite et ainsi réaliser l'obturation et le scellement du canal radiculaire.

Le ciment présente une dureté modérée lui permettant d'être repris de manière simple par exemple pour la réalisation d'une opération de restauration du ciment dentaire. Le ciment peut être retiré en tout ou partie du canal par mise en contact avec un outil vibrant à une fréquence ultrasonore, par exemple une pointe ultrasonique. La fréquence de vibration de l'outil peut être comprise entre 26 kHz et 36 kHz. L'outil vibrant à une fréquence ultrasonore est connu en soi et à titre d'exemple d'outil utilisable, on peut citer l'insert N°ET25S commercialisé par la société SATELEC.

Exemples

Le tableau ci-dessous fournit trois exemples de formulations de produits dentaires selon l'invention qui ont été préparés.

[Table 1]

| | | FORMULE A | FORMULE B | FORMULE C |
|---|---|---|---|---|
| Milieu liquide | Propylène glycol | 36,65% | 32,15% | 17,63% |
| | Eau | 0 | 0 | 17,50% |
| Liant | Kollidon 90F | 0,85% | 0,85% | 3,25% |
| Phosphates de calcium | DCPD | 15,75% | 17,50% | 17,50% |
| | TTCP | 29,25% | 32,50% | 32,50% |
| | Total phosphate de calcium | 45,00% | 50,00% | 50,00% |
| Agent de suspension | Aérosil® 200 | 0,50% | 1,00% | 0,63% |
| Agent de contraste radioopaque | Sulfate de baryum / Oxyde de zirconium | 10,00% | 10,00% | 10,00% |
| Agent antiseptique | Chlorhexidine | 1,00% | 1,00% | 1,00% |
| Accélérateur de prise | Hydroxyapatite | 6,00% | 5,00% | 0 |

Les constituants des formules A et B étaient conditionnés dans la même composition avant l'utilisation, alors que le phosphate dicalcique dihydraté (DCPD) et le phosphate tétracalcique (TTCP) étaient conditionnés dans deux compartiments distincts pour la formule C avec une distribution 1 :1 du contenu de ces compartiments au moment de l'utilisation pour former le produit dentaire de formule C. Les compositions dans chacun de ces deux compartiments sont fournies ci-dessous.

[Table 2]

| Composants | Pourcentage massique |
|---|---|
| Eau | 35,00% |
| DCPD | 35,00% |
| Kollidon 90F | 5,75% |
| BaSO4 | 20,00% |
| Aérosil® 200 | 0,25% |
| Chlorhexidine | 1,00% |
| Propylène glycol | 3,00% |

[Table 3]

| Composants | Pourcentage massique |
|---|---|
| Propylène glycol | 32,25% |
| TTCP | 65,00% |
| Kollidon 90F | 0,75% |

(suite)

| Composants | Pourcentage massique |
|---|---|
| Aèrosil 200 | 1,00% |
| Chlorexidine | 1,00% |

L'agent de contraste radio-opaque était le sulfate de baryum dans le cas des formules A et C et l'oxyde de zirconium dans le cas de la formule B. La taille moyenne D50 des grains du DCPD était de 10 μm (9,213 μm mesurée au granulomètre laser) et la taille moyenne D50 des grains du TTCP était inférieure à 10μm dans chacune des formules.

En milieu humide, la formule A a produit un ciment ayant une résistance à la compression de 3,27 MPa (la viscosité de la formule A était de 98,2613 Pa.s), la formule B un ciment ayant une résistance à la compression de 6,23 MPa (la viscosité de la formule B était de 142,127 Pa.s) et la formule C un ciment ayant une résistance à la compression de 4,34 MPa (la viscosité de la formule C était de 93,2593 Pa.s).

Les performances des ciments obtenus dans le cadre de l'invention ont été évaluées dans des essais de retraitement en aveugle sur des éprouvettes et des modèles de dents FKG remplis avec différents matériaux de scellement.

On a testé la formule B ci-dessus (B sur les photographies) ainsi que la formule A ci-dessus (C sur les photographies) en comparaison avec d'autres matériaux de scellement du commerce : « Total Fill BC Sealer » (A sur les photographies), « MTA Caps » (D sur les photographies) et « MTA Flow Extended Work Time » (« EWT » sur les photographies).

Avant l'essai de dégradation, la prise des matériaux de scellement a été vérifiée en soumettant une pression sur le dessus. L'ensemble des matériaux est dur et semble totalement pris. Les essais de dégradation ont été réalisés à l'aide du générateur à ultrasons Newtron P5XS à puissance 10 (puissance maximale de la gamme endodontie) et de l'insert ET25S, dédié à la reprise de traitement dans le tiers coronaire et les isthmes.

Lors des essais de dégradation sur les éprouvettes, les observations suivantes ont été réalisées :

- le ciment « Total Fill BC Sealer » est facilement dégradé à l'aide des ultrasons, il semble même être dissous au contact de l'eau (couleur blanchâtre de l'eau après le retraitement) ;
- les ciments obtenus à partir des formules selon l'invention sont, comme « Total Fill BC Sealer », facilement dégradés à l'aide des ultrasons. Il semble également qu'une dissolution soit présente mais de façon moins importante que pour « Total Fill BC Sealer » ;
- le ciment « MTA Flow EWT » est facilement dégradé à l'aide des ultrasons. Néanmoins, il n'est pas dissous au contact de l'eau ;
- le ciment « MTA Caps » est plus difficile à dégrader que les autres éprouvettes et montre une résistance plus importante. De plus, le matériau de scellement noircit au contact prolongé des ultrasons.

Lors des essais de dégradation des différents matériaux de scellement sur les modèles de dents FKG, des différences plus visibles sont apparues. En effet, sur des zones plus profondes et plus étroites, le ciment de scellement canalaire « Total Fill BC Sealer » présente une résistance plus importante qu'en éprouvette. La dégradation du matériau de scellement est possible petit à petit car il est impossible d'insérer l'insert profondément dans les canaux. Certaines parties semblent même plus difficiles à dégrader et nécessitent un temps de contact avec les ultrasons, prolongé. On note qu'avec ce matériau de scellement, la reprise de traitement est difficile dans les accès limités tels que les canaux étroits.

Les ciments obtenus dans le cadre de l'invention présentent une résistance réduite par rapport à « Total Fill BC Sealer ». En effet, au contact des ultrasons, il est très facile de dégrader l'ensemble du ciment et ce même dans les accès limités. On note également lors de cet essai que la formule contenant l'oxyde de zirconium possède une résistance légèrement plus importante que la formule contenant le baryum sulfate.

La reprise du traitement canalaire à l'aide des ultrasons est facilitée en présence des formules selon l'invention par rapport aux matériaux de scellement existants.

L'expression « compris(e) entre ... et ... » doit se comprendre comme incluant les bornes.

**Revendications**

1. Produit dentaire destiné à former un ciment endodontique dans un canal dentaire, ledit produit comprenant au moins :

- une composition pulvérulente en une teneur massique comprise entre 40 % et 55 % comprenant au moins un phosphate de calcium et apte à former de l'hydroxyapatite en présence d'humidité,
- un liant en une teneur massique comprise entre 0,5 % et 5 %, et
- un milieu liquide en une teneur massique comprise entre 30% et 50%.

2. Produit selon la revendication 1, dans lequel la teneur massique de la composition pulvérulente est comprise entre 43 % et 52 %.

3. Produit selon la revendication 1 ou 2, dans lequel la composition pulvérulente comprend un mélange de phosphate dicalcique et de phosphate tétracalcique.

4. Produit selon l'une quelconque des revendications 1 à 3, dans lequel la composition pulvérulente a une taille moyenne D50 de grains inférieure ou égale à 50 $\mu$m.

5. Produit selon l'une quelconque des revendications 1 à 4, dans lequel la teneur massique du liant est comprise entre 0,5 % et 3,5 %.

6. Produit selon l'une quelconque des revendications 1 à 5, comprenant en outre un agent de contraste radio-opaque en une teneur massique inférieure ou égale à 15%.

7. Produit selon l'une quelconque des revendications 1 à 6, comprenant en outre un agent de suspension en une teneur massique inférieure ou égale à 1,5%.

8. Produit selon l'une quelconque des revendications 1 à 7, comprenant en outre un accélérateur de prise du ciment en une teneur massique inférieure ou égale à 10%.

9. Produit selon l'une quelconque des revendications 1 à 8, dans lequel :

- la teneur massique en composition pulvérulente est comprise entre 43% et 52%,
- la teneur massique en liant est comprise entre 0,5% et 1%,
- le milieu liquide est non-aqueux et sa teneur massique est comprise entre 30% et 40%,
- le produit comprend un agent de contraste radio-opaque en une teneur massique comprise entre 5% et 15%, et
- le produit comprend un accélérateur de prise du ciment en une teneur massique comprise entre 4% et 7%.

10. Produit selon la revendication 9, dans lequel l'agent de contraste radio-opaque comprend du sulfate de baryum et dans lequel la teneur massique en composition pulvérulente est comprise entre 43% et 48%.

11. Produit selon la revendication 9, dans lequel l'agent de contraste radio-opaque comprend de l'oxyde de zirconium et dans lequel la teneur massique en composition pulvérulente est comprise entre 48% et 52%.

12. Produit selon l'une quelconque des revendications 1 à 8, dans lequel :

- la teneur massique en composition pulvérulente est comprise entre 43% et 52%,
- la teneur massique en liant est comprise entre 3% et 3,5%,
- le milieu liquide comprend de l'eau et sa teneur massique est comprise entre 30% et 40%, et
- le produit comprend un agent de contraste radio-opaque en une teneur massique comprise entre 5% et 15%.

13. Ensemble pour l'obturation et le scellement d'un canal dentaire, comprenant au moins le produit selon l'une quelconque des revendications 1 à 12 dans un dispositif d'introduction apte à introduire le produit dans le canal dentaire.


**Patentansprüche**

1. Dentalprodukt, das dazu bestimmt ist, einen endodontischen Zement in einem Wurzelkanal zu bilden, wobei das Produkt mindestens umfasst:

- eine pulverförmige Zusammensetzung mit einem Massenanteil von zwischen 40 % und 55 %, die mindestens ein Calciumphosphat umfasst und geeignet ist, bei Vorhandensein von Feuchtigkeit Hydroxylapatit zu bilden,

- ein Bindemittel mit einem Massenanteil von zwischen 0,5 % und 5 %, und
- ein flüssiges Medium mit einem Massenanteil von zwischen 30 % und 50 %.

2. Produkt nach Anspruch 1, wobei der Massenanteil der pulverförmigen Zusammensetzung zwischen 43 % und 52 % beträgt.

3. Produkt nach Anspruch 1 oder 2, wobei die pulverförmige Zusammensetzung ein Gemisch aus Dicalciumphosphat und Tetracalciumphosphat umfasst.

4. Produkt nach einem der Ansprüche 1 bis 3, wobei die pulverförmige Zusammensetzung eine mittlere Korngröße D50 von kleiner oder gleich 50 $\mu$m aufweist.

5. Produkt nach einem der Ansprüche 1 bis 4, wobei der Massenanteil des Bindemittels zwischen 0,5 % und 3,5 % beträgt.

6. Produkt nach einem der Ansprüche 1 bis 5, das ferner ein strahlenundurchlässiges Kontrastmittel mit einem Massenanteil von kleiner oder gleich 15 % umfasst.

7. Produkt nach einem der Ansprüche 1 bis 6, das ferner ein Suspensionsmittel mit einem Massenanteil von kleiner oder gleich 1,5 % umfasst.

8. Produkt nach einem der Ansprüche 1 bis 7, das ferner einen Abbindebeschleuniger für den Zement mit einem Massenanteil von kleiner oder gleich 10 % umfasst.

9. Produkt nach einem der Ansprüche 1 bis 8, wobei:

   - der Massenanteil an pulverförmiger Zusammensetzung zwischen 43 % und 52 % beträgt,
   - der Massenanteil an Bindemittel zwischen 0,5 % und 1 % beträgt,
   - das flüssige Medium nicht wässrig ist und sein Massenanteil zwischen 30 % und 40 % beträgt,
   - das Produkt ein strahlenundurchlässiges Kontrastmittel mit einem Massenanteil von zwischen 5 % und 15 % umfasst, und
   - das Produkt einen Abbindebeschleuniger für den Zement mit einem Massenanteil von zwischen 4 % und 7 % umfasst.

10. Produkt nach Anspruch 9, wobei das strahlenundurchlässige Kontrastmittel Bariumsulfat umfasst und wobei der Massenanteil an pulverförmiger Zusammensetzung zwischen 43 % und 48 % beträgt.

11. Produkt nach Anspruch 9, wobei das strahlenundurchlässige Kontrastmittel Zirkonoxid umfasst und wobei der Massenanteil an pulverförmiger Zusammensetzung zwischen 48 % und 52 % beträgt.

12. Produkt nach einem der Ansprüche 1 bis 8, wobei:

   - der Massenanteil an pulverförmiger Zusammensetzung zwischen 43 % und 52 % beträgt,
   - der Massenanteil an Bindemittel zwischen 3 % und 3,5 % beträgt,
   - das flüssige Medium Wasser umfasst und sein Massenanteil zwischen 30 % und 40 % beträgt, und
   - das Produkt ein strahlenundurchlässiges Kontrastmittel mit einem Massenanteil von zwischen 5 % und 15 % umfasst.

13. Anordnung zum Verschließen und zur Versiegelung eines Wurzelkanals, die mindestens das Produkt nach einem der Ansprüche 1 bis 12 in einer Einführungsvorrichtung umfasst, die geeignet ist, das Produkt in den Wurzelkanal einzuführen.

**Claims**

1. A dental product for forming an endodontic cement in a dental canal, said product comprising at least:

   - a powder composition in a mass content comprised between 40% and 55% comprising at least one calcium phosphate and able to form hydroxyapatite in the presence of moisture,
- a binder in a mass content comprised between 0.5% and 5%, and
- a liquid medium in a mass content comprised between 30% and 50%.

2. The product as claimed in claim 1, wherein the mass content of the powder composition is comprised between 43% and 52%.

3. The product as claimed in claim 1 or 2, wherein the powder composition comprises a mixture of dicalcium phosphate and tetracalcium phosphate.

4. The product as claimed in any one of claims 1 to 3, wherein the powder composition has an average particle size D50 of less than or equal to 50 $\mu$m.

5. The product as claimed in any one of claims 1 to 4, wherein the mass content of the binder is comprised between 0.5% and 3.5%.

6. The product as claimed in any one of claims 1 to 5, further comprising a radiopaque contrast agent in a mass content of less than or equal to 15%.

7. The product as claimed in any one of claims 1 to 6, further comprising a suspending agent in a mass content of less than or equal to 1.5%.

8. The product as claimed in any one of claims 1 to 7, further comprising a cement setting accelerator in a mass content of less than or equal to 10%.

9. The product as claimed in any one of claims 1 to 8, wherein:

   - the mass content of the powder composition is comprised between 43% and 52%,
   - the mass content of binder is comprised between 0.5% and 1%,
   - the liquid medium is non-aqueous and its mass content is comprised between 30% and 40%,
   - the product comprises a radiopaque contrast agent in a mass content comprised between 5% and 15%, and
   - the product comprises a cement setting accelerator in a mass content comprised between 4% and 7%.

10. The product as claimed in claim 9, wherein the radiopaque contrast agent comprises barium sulfate and wherein the mass content of the powder composition is comprised between 43% and 48%.

11. The product as claimed in claim 9, wherein the radiopaque contrast agent comprises zirconium oxide and wherein the mass content of the powder composition is comprised between 48% and 52%.

12. The product as claimed in any one of claims 1 to 8, wherein:

   - the mass content of the powder composition is comprised between 43% and 52%,
   - the mass content of binder is comprised between 3% and 3.5%,
   - the liquid medium comprises water and its mass content is comprised between 30% and 40%, and
   - the product comprises a radiopaque contrast agent in a mass content comprised between 5% and 15%.

13. A kit for filling and sealing a dental canal, comprising at least the product as claimed in any one of claims 1 to 12 in an introduction device suitable for introducing the product into the dental canal.

[Fig. 1]

[Fig. 2]

## RÉFÉRENCES CITÉES DANS LA DESCRIPTION

**Documents brevets cités dans la description**

- WO 2008102214 A